Europäisches Patentamt

European Patent Office

Office européen des brevets

(11) Publication number: **0 368 662 B1**

# EUROPEAN PATENT SPECIFICATION

(45) Date of publication of patent specification: **14.09.94** (51) Int. Cl.5: **C12N 5/24**

(21) Application number: **89311614.5**

(22) Date of filing: **09.11.89**

(54) **Parent cell lines for producing human hybridomas.**

(30) Priority: **09.11.88 JP 281460/88**
**09.11.88 JP 281461/88**

(43) Date of publication of application:
**16.05.90 Bulletin 90/20**

(45) Publication of the grant of the patent:
**14.09.94 Bulletin 94/37**

(84) Designated Contracting States:
**CH DE ES FR GB IT LI NL SE**

(56) References cited:
**EP-A- 0 182 467**
**US-A- 4 529 694**
**US-A- 4 720 459**
**US-A- 4 720 459**

**JOURNAL OF IMMUNOLOGICAL METHODS,
vol. 100, 1987, pages 5-40, Elsevier Science
Publishers B.V. (Biomedical Division); K.
JAMES et al.: "Human monoclonal antibody
production current status and future prospects"**

**Proceedings National Academy of Science,
USA 1979, 6651-6655**

(73) Proprietor: **MITSUI TOATSU CHEMICALS, Inc.
2-5 Kasumigaseki 3-chome
Chiyoda-Ku Tokyo 100 (JP)**

(72) Inventor: **Fukuda, Tamotsu
2142, Tougou
Mobara-shi Chiba-ken (JP)**
Inventor: **Okuya, Hiroaki
2142, Tougou
Mobara-shi Chiba-ken (JP)**
Inventor: **Kono, Naoka
90-1, Machibo
Mobara-shi Chiba-ken (JP)**
Inventor: **Kuroiwa, Yasuyuki
2791-1, Mutsuno
Mobara-shi Chiba-ken (JP)**

(74) Representative: **Harvey, David Gareth et al
Graham Watt & Co.
Riverhead
Sevenoaks Kent TN13 2BN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Monoclonal Antibody Production Techniques and Applications, L.B. Schook ed., New York, Marcel Dekker Inc. Chapter 1, "Practical Aspects of Human-Human Hybridomas", B.G. Campling et al, pp. 3-23

JOURNAL OF IMMUNOLOGICAL METHODS, Vol. 100, 1987, pages 5-40, Elsevier Science Publishers B.V.

**Description**

The present invention relates to novel parent cell lines for producing human hybridomas. More particularly, the present invention relates to novel parent cell lines for producing human hybridomas derived from human immunoglobulin synthesizing cells. A cell line according to the invention is itself incapable of producing human immunoglobulin and is capable of fusing with human antibody-producing cells. The cell line is selective to human hybridomas and is capable of imparting high antibody productivity to human hybridomas.

In 1975, Köhler and Milstein selected and isolated for the first time a single mouse hybridoma capable of producing mouse monoclonal antibody by using mouse myeloma cell line P3X63Ag8 as a parent cell line, fusing the parent cells with mouse spleen cells and then culturing the fused cells in selective culture medium containing aminoputerine, hypoxanthine and thymidine [G. Köhler and C. Milstein, Nature, 256, 495 (1975)]. Since then attempts have been made to produce a single human hybridoma capable of producing human monoclonal antibody by using as the parent cell line a mouse myeloma cell line, a hetero myeloma cell line, which is a hybridoma between mouse myeloma cell and human cell, a human myeloma cell line or a human lymphoblast cell line and fusing the parent cell line with human antibody-producing cell. However, in the case of using as the parent cell line a mouse myeloma cell line or a hetero myeloma cell line and fusing the parent cell line with a human antibody-producing cell, the thus produced human hybridomas also synthesize and secret mouse protein (an unwanted antigen) in addition to human antibodies. Therefore, it is not necessarily appropriate to use the resulting hybridomas for producing human monoclonal antibodies for administration to humans.

On the other hand, an attempt to produce human hybridomas by fusion between a parent cell line having human chromosome alone and a human antibody-producing cell has been reported by Olsson and Kaplan as well as Croce et al. in 1980 [L. Olsson and H.S. Kaplan, Proc. Natl. Acad. Sci., 77 5429 (1980), C.M. Croce et al., Nature 288, 488 (1980)]. Then many reports on the production of hybridomas followed. However, in parent cell lines suited for producing human hybridomas capable of imparting high antibody productivity to the resulting human hybridomas, the cells themselves synthesize human immunoglobulin, though its degree is different. For example, KR-12 (disclosed in EPA 182467) having selectivity to a human hybridoma between human myeloma cell line RPMI 8226 and human lymphoblast cell line KR-4 is currently one of a few parent cell lines suited for producing human hybridomas, however it senthesizes and secretes heavy chain (human gamma chain) and light chain (human lambda chain, human kappa chain) of immunoglobulins derived from the respective cell lines [Japanese Patent Laying-Open No. 61-128886] (a Japanese patent laying-open means the publication of an application). As parent cell lines for producing human hybridomas derived from human myeloma cell lines or human lymphoblasts, several patent applications have been filed, directed to ATCC CRL 8032 and ATCC CRL 8038 [Japanese Patent Laying-Open No. 57-126424], WI-L2-729 HF2 [Japanese Patent Laying-Open No. 57-208987], ATCC CRL 8083 [Japanese Patent Laying-Open No. 58-501257], ATCC CRL 8147 [Japanese Patent Laying-Open No. 59-66883], UC 729-6 [U.S. Pat. No. 4,451,570], ATCC CRL 8221 [Japanese Patent Laying-Open No. 59-198970], LTR228 [ Japanese Patent Laying-Open No. 60-251881], HIH/TOI[ Japanese Patent Laying-Open No. 62-155083], and ATCC HB 9320 [Japanese Patent Laying-Open No. 64-60373]. Some of these parent cell lines do not secrete human immunoglobulin but all of them synthesize human immunoglobulin.

For this reason, attempts have been made to mutate a cell line incapable of producing human immunoglobulin from the existing parent cell line suited for production of human hybridomas. For example, "Monoclonal Antibody Production Techniques and Applications" authored and edited by L.B.S. Chook (1987), MARCEL DEKKER, INC., page 12, describes isolating cells incapable of expressing human immunoglobulin on the cell surface by reacting anti-human immunoglobulin antibody with a cell mass mutated from KR-12 in the presence of a complement to kill cells expressing human immunoglobulin on the cell membrane surface followed by cloning manipulation using cell sorting and limiting dilution. However, cells incapable of synthesizing human immunoglobulin could not be obtained.

As a parent cell line incapable of producing human immunoglobulin, a patent application has been filed directed to HOMO7 [Japanese Patent Laying-Open No. 63-185374]. However, no working example is found on the amount of antibodies secreted by human hybridoma produced. The presence of a parent cell line derived from cells other than human myeloma cells or human lymphoblasts or their human hybridomas is also reported. However, there is no description of the amount of antibodies secreted by human hybridomas produced in patent applications directed to Burkitt's lymphoma-derived parent cell lines [Japanese Patent Laying-Open Nos. 60-141285 and 61-242575]. In a patent application directed to a human melanoma cell-derived parent cell line [Japanese Patent Laying-Open No. 59-132885], no working example is found on the production of human hybridomas.

EP 0 368 662 B1

USA 4,720,459 discloses a mutant human cell line which does not secrete immunoglobulin. In such cells containing a block in secretion rather than synthesis, the possibility of recombined antibody generation still exists. USA 4,529,694 discloses a human cell line which lacks a marker for drug resistance and which cannot therefore be used as a parent cell line.

Where the yield of antibodies produced from the human hybridomas produced is low, costs for producing of antibodies are high, so that industrial utilization is generally considerably limited. For this reason, a parent cell line that cannot impart high antibody productivity to the desired human hybridoma so produced is of little value for industrial utilization.

On the other hand, where a parent cell line itself synthesizes human immunoglobulin, the resulting human hybridoma synthesizes human immunoglobulin derived from human antibody-producing cells and human immunoglobulin derived from the parent cell line. Therefore, there is a possibility that, on occasion, a plurality of partially recombined antibodies might be secreted. In addition, the binding specificity of the antibody to antigen is derived from the structure of both heavy chain and light chain of human immunoglobulin in the variable amino acid sequence region. Accordingly, where the heavy chains and light chains derived from human antibody-producing cells and the parent cell line are recombined with each other, it is presumed that the binding activity or specificity of the secreted antibodies to the desired antigens might be reduced. In fact, Shinmoto et al. reported that the human hybridoma between the parent cell line HO323 capable of synthesizing but incapable of secreting human IgM and human lymphocyte produces the antibody in which the heavy chain and light chain of human IgM derived from the parent cell line are recombined with the heavy chain and light chain of human IgA derived from the human lymphocyte [H. Shinmoto et al., Agric. Biol. Chem., 50, 2217 (1986)].

It was reported that the human hybridoma between KR-12 and human IgM-producing cell line having reactivity with tetanus toxin secreted almost the same amount of human IgG and human IgM and furthermore, human IgM alone out of the two antibodies has a reactivity with tetanus toxin [D. Kozbor et al., J. Immunol., 133, 3001 (1984)].

The present inventors have made extensive investigations in an attempt to produce a parent cell line derived from human for human hybridomas imparting high antibody productivity to the produced human hybridomas yet the cell per se being incapable of synthesizing human immunoglobulin. As a result, we have successfully isolated a novel parent cell line for human hybridoma which is a mutant cell line derived from a human hybridoma capable of synthesizing and secreting heavy chain (human gamma chain) and light chain (human lambda chain, human kappa chain) of human immunoglobulin, which novel parent cell line does not synthesize heavy chain (human gamma chain), does not synthesize heavy chain (human gamma chain) and light chain (human lambda chain) or, does not synthesize heavy chain (human gamma chain) and light chain (human lambda chain, human kappa chain) of human immunoglobulins.

Köhler reported that a mouse hybridoma capable of secreting a plurality of different antibodies was produced and the mechanism on deletion of expression of the heavy chain and light chain in mouse immunoglobulin was examined. As the result, deletion in expression of the heavy chain of mouse immunoglobulin was noted mainly due to chromosomal deficiency [G. Köhler, Proc. Natl. Acad. Sci., 77, 2197 (1980)]. On the other hand, the present inventors found in the past that when a mouse hybridoma between a mouse myeloma cell and a mouse spleen cell was repeatedly freeze stored and subcultured continuously, mouse hybridomas maintaining a high proliferation characteristics as myeloma cells are expressed with high frequency, notwithstanding that they lost the ability to synthesize and secrete antibodies.

Therefore, after inducing mutation on the known and generally available human hybridoma cell line ATCC CRL 8658, a novel single cell line was produced by screening cells that maintain the proliferation ability but do not synthesize or secrete the heavy chain (human gamma chain) of human immunoglobulin or, both or either one of the heavy chain (human gamma chain) and light chain (human lambda chain, human kappa chain) of human immunoglobulin. We have confirmed that this novel cell line is capable of fusing with human antibody-producing cells, maintains the selectivity characteristic of human hybridomas and, unlike the parent cell line used to induce the present parent cell line, this human hybridoma synthesizes and secretes human immunoglobulin derived from the human antibody-producing cells. It has thus been found that the present parent cell line is a parent cell line useful for the production of human hybridomas suited for practically and efficiently producing human monoclonal antibodies.

What is particularly important in the present invention is the successful isolation of a parent cell line that does not synthesize the heavy chain (human gamma chain), does not synthesize the heavy chain (human gamma chain) and the light chain (human lambda chain), or does not synthesize the heavy chain (human gamma chain) and the light chain (human lambda chain, human kappa chain), as the result of mutation for the purpose of obtaining a cell line from which only the undesired characteristic that the parent cell line

4

itself synthesizes human immunoglobulin although it maintains the ability of imparting high antibody productivity to the human hybridoma with human antibody-producing cells. It is further been confirmed that the novel parent cell line of this invention is capable of fusing with human lymphoblasts, can impart high human antibody productivity to the exemplified human hybridomas and can produce only the heavy chain derived from human lymphoblasts as the heavy chain.

Therefore, an object of the present invention is to provide a parent cell line for producing a cell line capable of industrial production of human monoclonal antibodies which can be used over wide areas such as prophylaxis, treatment, diagnosis, etc. of various diseases.

An important feature of the present invention lies in producing cells themselves incapable of synthesizing human immunoglobulin from cells that are capable of synthesizing human immunoglobulin. The starting materials are limited to a particular human hybridoma cell line ATCC CRL 8658 between human myeloma cell line RPMI 8226 and human lymphoblast cell line GM 1500.

According to the present invention there is provided a human $\gamma$-chain-non-synthesizing mutant cell line derived from human immunoglobulin-synthesizing cell line ATCC CRL 8658, and suitable for use as a fusion partner for making human hybridomas capable of synthesizing specific monoclonal antibodies, and a functionally-equivalent cell line derived therefrom.

The invention also provides a mutant cell line of Deposit No. FERM BP-2128, FERM BP-2129 or FERM BP-2615, and a functionally-equivalent cell line derived therefrom.

The invention further provides human hybridomas obtained by cell fusion between the mutant cell lines defined in the preceding two paragraphs and human antibody-producing cells; it provides also monoclonal antibodies from these hybridomas.

The invention will now be explained in more detail in the following description, which is given by way of illustrative example only.

The novel immunoglobulin-non-producing mutant of the present invention can be obtained by producing a mass of mutants through manipulation for mutation, such as variation or deletion of the chromosome from known human hybridomas, e.g., KR-12, etc., screening from the cell mass the presence of cells of which all or a part of the capability of producing human immunoglobulin is deleted and isolating a single cell line through cloning. Furthermore, by allowing again to cause mutation on the cells during the course of cloning or on the isolated single cell line and repeating cloning, a single cell line of which all or a part of the capability of producing human immunoglobulin is deleted can be isolated. Finally by adapting to a medium containing 8-azaguanine and ouabain, the novel immunoglobulin-non-producing mutant can be produced.

As the human immunoglobulin-producing cell line used in the present invention, there are, in addition to the known cell lines such as KR-12, etc. described above, a variety of human myeloma cells, human lymphoblasts or human hybridomas newly produced by fusion of these cells. KR-12 used in the present invention is available from ATCC (American Type Culture Collection) as CRL 8658.

Mutation for variation or deletion of the chromosome can be performed by environmental change through manipulation such as a plurality of operations for freezing and thawing, overdensity culture, low oxygen concentration culture, low temperature or high temperature culture, change in culture temperature in a short period of time, etc., or by irradiating ultraviolet rays, radiation, etc. in a pulse interval or continuously. The mutation can also be effected by adding mutagenic chemicals such as 8-azaguanine, 6-thioguanine, colchicine, ethylmethane sulfonate (EMS), N-methyl-N'-nitro-N-nitrosoguanidine (MNNG), acridine mastard, etc. to a medium in a definite amount. These methods are applied singly or in appropriate combination to effectively induce variation or deletion of the chromosome.

Cell culture can be carried out by dispersing in a medium in a cell density of, e.g., $5 \times 10^4$/ml to $2 \times 10^6$/ml, inoculating the culture solution on an appropriate cell culture vessel and culturing at 37°C in the presence of 5% carbon dioxide gas. Preferred examples of the medium include those obtained by supplementing basal medium such as RPMI 1640, Dulbecco modified Eagle's medium (DMEM), etc. with a suitable amount of fetal calf serum (FCS). In addition, various serum-free media can also be used. For example, NYSF 404 serum-free medium supplemented with an appropriate amount of bovine serum albumin is recommended. In subculture, it is preferred to repeat the operation of cell recovery and inoculation in an interval of 3 to 7 days.

Freezing of the cells can be carried out in a conventional manner. For example, the cells are dispersed in a spitable frozen cell storage solution in a cell density of $1 \times 10^5$/ml to $5 \times 10^7$/ml and the dispersion is frozen and stored in liquid nitrogen or liquid nitrogen gas or in a refrigerator at -20 to -80°C. It is recommended to use as the frozen cell storage solution one prepared by adding a suitable amount of animal serum or albumin, glucose, dimethyl sulfoxide (DMSO), etc. to the basal medium described above, neutral buffer solution, etc.

Operations for thawing the frozen cells, treatment after thawing and reincubation can be performed in a conventional manner. For example, the frozen cells are rapidly thawed in warm water. After the thawing, the cells are washed with a suitable medium to wash off DMSO contained in the storage solution. Then, the cells are dispsersed in a medium followed by incubation.

Cloning of the cell line of the present invention from the mutants can be performed by the soft agar method ["Studies on Applied Tissue Culture", page 289 (1985) published by Soft Science Co., Ltd., etc.] or the limiting dilution method ["Monoclonal Antibody", page 73 (1983), published by Kodansha Publishing Co., Ltd., etc.]. In cloning according to, e.g., the limiting dilution method, the cell mass on which mutation was induced is dispersed in a medium containing 20% FCS and the dispersion is inoculated on a 96 well flat bottom plate on which mouse spleen cells are inoculated as feeder cells (hereafter simply referred to as the feeder plate) in one per well followed by culturing at 37°C in the presence of 5% carbon dioxide gas. With respect to the well in which proliferation is noted as a single colony, the amount of human immunoglobulin in the culture supernatant is subjected to screening. With respect to the cells in the well in which production of human immunoglobulin is not noted, cloning is again performed by the limiting dilution method. By repeating the cloning operation several times, a cell line secreting no human immunoglobulin can be obtained as a single cell mass. By adding a chemical that kills the cells with hypoxanthine phosphate ribosyl transferase (hereafter simply referred to as HGPRT) to the medium in cloning, HGPRT-deficient cells can be efficiently selected. As such a chemical, 8-azaguanine or the like is preferred.

Screening of human immunoglobulin in the culture supernatant can be performed by conventional radioimmunoassay or enzyme-linked immunosorbent assay (ELISA), etc. In the case of using, e.g., ELISA, anti-human immunoglobulin antibody is immobilized onto a solid phase (the antibody used in this case is hereafter simply referred to as immobilized antibody) and a part of the culture supernatant is reacted with it. Next the reaction product is reacted with enzyme-labeled anti-human immunoglobulin antibody and substrate is added thereto. By measuring the color formed by the enzyme reaction, human immunoglobulin in the culture supernatant can be detected and the quantity of human immunoglobulin can be determined. Determination of human gamma chain, human lambda chain or human kappa chain can be carried out by using as the immobilized antibody anti-human IgG (human gamma chain-specific) antibody, anti-human lambda chain antibody or anti-human kappa chain antibody, and using as the enzyme-labeled antibody peroxidase-labeled anti-human IgG (human gamma chain-specific) antibody, peroxidase-labeled anti-human lambda chain antibody or peroxidase-labeled anti-human kappa chain antibody, respectively.

The parent cell line of the present invention is dead in a medium containing hypoxanthine, aminoputerine and thymidine (HAT medium) or in a medium containing hypoxanthine and azaserine (HA medium). This HGPRT-deficient property can be maintained by culturing in a medium containing 8-azaguanine in a concentration of 10 $\mu$g/ml to 100 $\mu$g/ml. Furthermore, the parent cell line is not dead in a medium containing ouabain in a concentration of 10 $\mu$M. Based on this selectivity property, human hybridomas between the parent cell line of the present invention and human antibody-producing cells can be produced. Furthermore, the resulting human hybridomas can secrete only the antibodies substantially derived from the human antibody-producing cells into the medium.

The parent cell line of the present invention can be subcultured using the medium described above. The parent cell line of the present invention can also be stably stored using the storage solution described above over a long period of time.

Fusion between the cells of the present invention and human antibody-producing cells can be carried out using conventional chemicals for fusion such as polyethylene glycol (hereafter simply referred to as PEG) or viral particles such as Sendai virus (Hemagglutinating Virus of Japan: HVJ), etc. For example, a solution obtained by adding PEG having a mean molecular weight of approximately 1000 to 6000 to RPMI 1640 medium or DMEM medium in a concentration of 30 to 50% (W/V) is preferred as a fusing solution. In order to further enhance a fusion efficiency, it is also preferable to add DMSO to the fusing solution. The fusion can also be effected by physical means using an electrofusion apparatus, etc. In the cell fusion, it is desired to use the human antibody-producing cells in an amount of 1 to 10 times that of the cells of the present invention. As the human antibody-producing cells, a cell mass transformed by Epstein-Barr virus (hereafter simply referred to as EB virus) and a single EB virus transformant obtained from the transformed cell mass by cloning can be used. In addition, lymphocyte fraction and human B cell fraction containing human antibody-producing cells derived from human antibody-producing cells obtained by stimulation and proliferation of these fractions with mitogen or antigen, etc. can be used as the human antibody-producing cells for cell fusion.

By the methods described above, the human antibody-producing cells are fused with the cells of the present invention. The fused cells are separately charged onto a culture plate of 24 wells or 96 wells followed by culturing in the selection medium at 37°C in the presence of 5% carbon dioxide gas. During the culture, it is preferred to exchange half of the selection medium with a fresh selection medium at 3 to 5 day intervals. In this case, when mouse peritoneal exudate cells or the like are allowed to co-exist as the feeder cells, proliferation of human hybridomas can be accelerated. From the cell mass, the human hybridomas can be selected by the selection media. If the human antibody-producing cells have no infinite limiting proliferation ability, HAT medium or HA medium can be used as the selection medium. When the human antibody-producing cells have infinite proliferation ability like EB virus transformants, etc., HAT medium or HA medium containing ouabain can be used as the selection medium.

The present invention is now described in more detail by referring to the following non-limiting examples.

Example 1. Production of human immunoglobulin-non-producing cell line 1:

(1) Production of the mutant cell line

Human hybridoma ATCC CRL 8658 was suspended in 10 ml of RPMI 1640 medium containing 10% FCS (hereafter simply referred to as 10% FCS culture medium) in a density of $1 \times 10^5$ cells/ml. The suspension was inoculated on a plastic culture flask having a bottom area of 75 $cm^2$ (75T flask, Coaster) followed by stationary culture at 37°C for 5 days in the presence of 5% carbon dioxide gas.

The culture was transferred to a plastic centrifuging tube having a 15 ml volume (Corning). The cells were collected by centrifugation (200 x g, 10 minutes) and suspended in RPMI 1640 medium containing 75% FCS and 10% DMSO (hereafter simply referred to as freeze storage solution) in a density of $1 \times 10^6$ cells/ml. After 1 ml/tube of the cell suspension was separately charged in a stock tube (Corning) having a 2 ml volume, the suspension was settled at -20°C for an hour to freeze and were then stored at -80°C.

The frozen cell suspension in the stock tube was thawed while gently stirring in a hot bath of 37°C. The thawed cell suspension was transferred to a plastic centrifuging tube having a 15 ml volume and washed twice with 10 ml of 10% FCS culture medium. After inoculation on a 75T flask, stationary culture was carried out. The foregoing operations for cell culture (5 days) and freeze storage (2 days) were continuously performed over 6 weeks.

The frozen cell suspension was thawed and the cells were washed twice with 10 ml of 10% FCS culture medium. Thereafter, the cells were suspended in 10 ml of 10% FCS culture medium in a density of $5 \times 10^4$ cells/ml. After inoculation on a 75T flask, stationary culture was carried out for 4 days. The cells were collected by centrifugation and suspended in 1 ml of 10% FCS culture medium containing EMS of a concentration of 200 $\mu$g/ml. After inoculation on one well of a 6 well culture plate (Coaster), stationary culture was carried out for 24 hours. The culture was centrifuged and washed twice with 10 ml of 10% FCS culture medium. Thereafter the cells were suspended in 12 ml of 10% FCS culture medium. After inoculation on a 75T flask, stationary culture was carried out for 4 days. The culture was centrifuged and washed twice with 10 ml of 10% FCS culture medium to give $6.0 \times 10^5$ of cells. The cells were suspended in 6 ml of 10% FCS culture medium. After inoculation on a 75T flask, stationary culture was carried out for 3 days. The culture was centrifuged to give $7.0 \times 10^5$ of mutation-induced cells.

(2) Screening and cloning

The mutation-induced cells were suspended in 20% FCS culture medium containing 15 $\mu$g/ml of 8-azaguanine (Tokyo Chemical Co., Ltd.) in a density of 10 cells/ml and 0.1 ml each of the suspensions was inoculated on each well of 10 plates of a 96 well flat bottom culture plate (Corning) on which $1 \times 10^5$ of mouse spleen cells had been previously inoculated as feeder cells followed by stationary culture. Two or three weeks after, the culture supernatant was collected in order from the wells in which colonies had sufficiently proliferated as a single clone. The presence or absence of human immunoglobulin in the culture supernatant was determined by screening in accordance with ELISA using a 96 well flat bottom plate (Greiner) for EIA and using goat anti-human immunoglobulin antibody (Tago) as immobilized antibody solution and peroxidase-labeled goat anti-human immunoglobulin antibody (Tago) as enzyme-labeled antibody. As the result, human gamma chain was not detected in the culture supernatant in ten wells (1C3, 1G10, 3F6, 3H2, 4B11, 5C5, 7A8, 8D1, 8H7 and 10F8) out of the 250 wells in which cell growth was noted.

The cells in the ten wells were independently collected and suspended in 20% FCS culture medium in a density of 10 cells/ml. After inoculating 0.1 ml each on each well of a feeder plate in one plate per one cell line, stationary culture was carried out. Two or three weeks after, the culture supernatant was collected in order from the wells in which colonies had proliferated as a single clone. The production of human gamma chain in the culture supernatant was determined by screening in accordance with ELISA. As the result, the production of human gamma chain was not detected in all of the culture supernatant in 68 wells in which colonies were proliferated as a single clone, in the plate inoculated with 7A8. With respect to the cells in the wells in which the production of human gamma chain was not noted, similar cloning operations were again repeated to give one single cell line. The cell line was named MP 4109. MP 4109 has been deposited under the terms of the Budapest Treaty in Fermentation Research Institute of the Agency of Industrial Science and Technology of Japan as Deposit Number FERM BP-2129.

Example 2. Production of human immunoglobulin-non-producing cell line 2:

(1) Production of the mutant cell line

The cells in ten wells (1C3, 1G10, 3F6, 3H2, 4B11, 5C5, 7A8, 8D1, 8H7 and 10F8) from which no human gamma chain was detected in Example 1 were collected and suspended in 5 ml of 20% FCS culture medium. The suspension was inoculated on a culture flask having a bottom area of 25 cm$^2$ (25T flask, Corning) followed by stationary culture for 4 days. The cells were collected by centrifugation to give 3 x 10$^6$ cells. The cells were suspended in 3 ml of 10% FCS culture medium containing MNNG of a concentration of 0.5 $\mu$g/ml and 1 ml each of the suspension was separately charged in 3 wells of a 6 well culture plate followed by stationary culture for 24 hours. The cultures in the 3 wells were centrifuged and washed twice with 10% FCS culture medium to give 3.2 x 10$^6$ cells, from which 1 x 10$^6$ cells were suspended in 10 ml of 10% FCS culture medium. After inoculation on a 75T flask, stationary culture was carried out for 2 days. The culture was centrifuged to give 1.2 x 10$^6$ cells. The cells were suspended in 10 ml of 10% FCS culture medium. After inoculation on a 75T flask, stationary culture was carried out for 2 days. The culture was centrifuged to give 5.5 x 10$^5$ cells. The cells were suspended in 5 ml of 10% FCS culture medium. After inoculation on a 25T flask, stationary culture was carried out for 2 days. The culture was centrifuged to give 2.5 x 10$^5$ cells. The cells were suspended in 2 ml of 10% FCS culture medium and 1 ml each of the suspension was separately charged in 2 wells of a 6 well culture plate followed by stationary culture for 2 days. The culture was centrifuged to give 7.0 x 10$^5$ of mutation-induced cells.

(2) Screening and cloning

The mutation-induced cells were washed once with 10% FCS culture medium and suspended in 10% FCS culture medium in a density of 40 cells/ml. 0.1 ml each of the suspension was inoculated on each well of 4 plates of 96 1/2 well flat bottom culture plate (Corning) on which 1 x 10$^5$ of mouse spleen cells had been previously inoculated as feeder cells followed by stationary culture for 15 days. During the culture, half of the medium was exchanged with a fresh medium at 3 to 4 day intervals. The culture supernatant was collected from the wells in which colonies had sufficiently proliferated as a single clone. The presence or absence of human immunoglobulin in the culture supernatant was determined by screening in accordance with ELISA.

With respect to the culture supernatant in 285 wells in which colonies were proliferated as a single clone, the quantity of human immunoglobulin was determined. As the result, the production of human gamma chain, human lambda chain and human kappa chain was negative in the culture supernatant from 4 wells (S1A6, S2C10, S2G7 and S4D3); the production of human gamma chain and human lambda chain was negative in the culture supernatant from 2 wells (S2E7, S4E2); and the production of human gamma chain and human kappa chain was negative in the culture supernatant from 1 well (S1F5). The cells in each well were cultured on an enlarged scale and the quantity of human immunoglobulin in the resulting culture supernatant was again determined. As the result, the culture supernatant in the well on which the cells in S4D3 were inoculated showed negative production of human kappa chain and the culture supernatant in the well on which the cells in S2E7 were inoculated showed negative production of human lambda chain. Next, the cells in S2E7 were suspended in 10% FCS culture medium in a density of 20 cells/ml and again cloned (using 4 feeder plates). Sixteen days after inoculation, with respect to the wells in which colonies had sufficiently proliferated as a single clone, the presence or absence of human immunoglobulin in the culture supernatant was determined by screening in accordance with ELISA. The production of human gamma chain and human lambda chain was negative in the culture supernatant from 17 wells (1D3, 2A9, 2F7, 4B2, etc.) out of 23 wells assayed. The next day, the cells in 4B2 were cloned (using 3 feeder plates). Sixteen days after inoculation, with respect to the wells in which colonies were sufficiently proliferated as a single clone, the presence or absence of human immunoglobulin in the culture supernatant was determined by screening in accordance with ELISA. The production of human kappa chain was positive but the production of human gamma chain and human lambda chain was negative, in all of the culture supernatants from the assayed 281 wells. From them, a single cell line having excellent proliferation was selected and named MP 4112. MP 4112 has been deposited under the teas of the Budapest Treaty in Fermentation Research Institute of the Agency of Industrial Science and Technology of Japan as Deposit Number FERM BP-2128.

Example 3. Production of human immunoglobulin-non-producing cell line 3:

(1) Production of the mutant cell line

The cell line incapable of synthesizing human gamma chain and human lambda chain of immunoglobulins was isolated from human hybridoma ATCC CRL 8658 in a manner similar to Example 1. The $5 \times 10^5$ cells of this cell line were suspended in 5 ml of 10% FCS culture medium containing EMS of a 150 $\mu$g/ml concentration. The suspension was inoculated on a 25T flask followed by stationary culture for 24 hours. The cells were collected by centrifugation to give mutation-induced cells.

(2) Screening and cloning

The mutation-induced cells were washed twice with 10% FCS culture medium and suspended in a density of $1.2 \times 10^6$ cells/ml. 0.1 ml of the suspension was added to and mixed with 24 ml of the 20% FCS culture medium containing 0.3% agarose (Sea Plaque agarose, F.M.C. Inc.). Next, 3 ml of the culture medium containing cells were charged and solidified on 6 cm Petri dishes (7 dishes) which had been previously solidified by charging 4 ml each of the 16.7% FCS medium containing 0.5% agarose.
The 6 cm Petri dish charged with the cells was subjected to stationary culture at 37°C in the presence of 5% carbon dioxide gas. Two weeks after, the cells were proliferated in soft agar and colones were observed with the naked eye. Each colony was transferred to each well of a 96 well flat bottom culture plate, in which 0.1 ml per well of the 20% FCS medium had previously been charged, using a Pasteur pipette. Two days after, 0.1 ml of the 20% FCS medium was supplemented and a further 3 days after, the culture supernatant was collected. The presence or absence of human kappa chain in the culture supernatant was determined by screening using ELISA.
With respect to the culture supernatant in 432 wells, the quantity of human kappa chain was determined. As the result, the production of human kappa chain was negative in the supernatant from 4 wells (2F10, 2G9, 3E9, 4H7). The cells in each well were cultured on an enlarged scale and the quantity of human kappa chain in the resulting culture supernatant was again determined. As the result, the culture supernatant in the well on which the cells in 2G9 were inoculated showed negative production of human kappa chain. Next, the cells in 2G9 were suspended in 10% FCS culture medium in a density of 40 cells/ml and inoculated on each well of 3 plates of 96 1/2 well flat bottom culture plate on which $1 \times 10^5$ of mouse spleen cells had been previously inoculated as feeder cells followed by stationary culture for 8 days. Nine days after inoculation, with respect to the wells in which colonies were sufficiently proliferated as a single clone, the presence or absence of human immunoglobulin in the culture supernatant was determined by screening in the accordance with ELISA. The production of human kappa chain was negative, in all of the culture supernatants from the assayed 184 wells. From them, a single cell line having excellent proliferation was selected and named MP 4126. MP 4126 has deposited under the terms of the Budapest Treaty in Fermentation Research Institute of the Agency of Industrial Science and Technology of Japan as Deposit Number FERM BP-2615.

Example 4. Measurement of Chromosomal Count

Distribution in the chromosomal count of MP 4109, MP 4112 and MP 4126 was determined in a manner similar to the Yoshida method ["Practical Manual for Utilization of Animal Cells", page 337 (1984), Realize Co., Ltd.].
MP 4109, MP 4112 and MP 4126 in the log phase were collected and approximately $1 \times 10^6$ cells were cultured for 90 minutes in 10% FCS culture medium containing 0.1 $\mu$g/ml of colcemid (Sigma). After culture, centrifugation was performed (200 x g, 5 minutes) and 5 ml of 0.075 M potassium chloride aqueous solution was added to the cell residue. The mixture was gently agitated and allowed to stand at 37°C for 20 minutes. After allowing it to stand, 1 ml of a fixing fluid (Carnoy's solution; methanol : acetic acid = 3 : 1) was added and the mixture was gently agitated followed by allowing it to stand at 4°C for 30 minutes. Next, centrifugation was performed (180 x g, 5 minutes) and 3 ml of the culture supernatant was removed. The same amount of fixing solution was added followed by agitation. The amounts of the culture supernatant to be removed after centrifugation increased to 4, 5 and 6 ml, repeating the same operations. Lastly, 0.5 ml of

9

fixing solution was added to the cell residue to form a suspension. Several drops of the suspension were dropped onto a glass slide. After allowing it to stand on a boiling water bath for about 1 minute, the glass slide was dried in the air at room temperature.

After the glass slide was immersed for 15 minutes in Gimsa solution diluted with 1/15 M phosphate buffer (pH 7.0) to 20-fold, superfluous staining solution was washed out with tap water followed by drying. After drying, an inclusion chemical was added dropwise and the glass slide was covered with a cover glass. A picture was taken under an optical microscope to count chromosomes. As shown in Tables 1, 2 and 3, MP 4109, MP 4112 and MP 4126 had modes on 89, 75 and 73 counts of chromosome, respectively.

Table 1.  Distribution of chromosome in MP 4109

| Number of Sample | 1 | 6 | 2 | 9 | 3 | 4 | 5 | 13 | 15 | 10 | 10 | 16 | 6 | 6 | 1 | 3 | 1 |
|---|---|---|---|---|---|---|---|---|---|---|---|---|---|---|---|---|---|
| Count of Chromosome | 78 | 79 | 80 | 81 | 82 | 83 | 84 | 85 | 86 | 87 | 88 | 89 | 90 | 91 | 92 | 93 | 100 |

Total number of samples = 126

Table 2.  Distribution of chromosome in MP 4112

| Number of Sample | 1 | 2 | 1 | 1 | 1 | 1 | 1 | 2 | 2 | 1 | 2 | 2 | 1 | 4 | 7 | 5 | 3 | 10 |
|---|---|---|---|---|---|---|---|---|---|---|---|---|---|---|---|---|---|---|
| Count of Chromosome | 51 | 56 | 58 | 59 | 61 | 62 | 63 | 64 | 65 | 67 | 68 | 69 | 70 | 71 | 72 | 73 | 74 | 75 |

| Number of Sample | 8 | 8 | 3 |
|---|---|---|---|
| Count of Chromosome | 76 | 77 | 78 |

Total number of samples = 66

Table 3.  Distribution of chromosome in MP 4126

| Number of Sample | 1 | 1 | 1 | 2 | 1 | 1 | 1 | 2 | 2 | 2 | 1 | 2 | 3 | 2 | 6 | 2 | 4 | 1 |
|---|---|---|---|---|---|---|---|---|---|---|---|---|---|---|---|---|---|---|
| Count of Chromosome | 48 | 55 | 56 | 57 | 61 | 63 | 64 | 65 | 66 | 68 | 69 | 70 | 71 | 72 | 73 | 74 | 76 | 77 |

| Number of Sample | 1 | 1 | 2 | 1 | 4 | 3 | 3 | 1 | 1 | 1 | 1 | 1 | 1 | 1 | 1 | 1 | 1 |
|---|---|---|---|---|---|---|---|---|---|---|---|---|---|---|---|---|---|
| Count of Chromosome | 78 | 79 | 80 | 81 | 82 | 83 | 84 | 85 | 87 | 90 | 96 | 97 | 104 | 109 | 112 | 125 | 127 |

| Count of Chromosome | 1 | 1 |
|---|---|---|
| Number of Sample | 134 | 150 |

Total number of samples = 62

Example 5. Measurement of human immunoglobulin synthesized and secreted

A sample for measuring the amount of human immunoglobulin secreted out of the cells by MP 4109, MP 4112 and MP 4126 was prepared as follows. The cells in the log phase were collected and suspended in 10% FCS culture medium in a density of $1 \times 10^6$ cells/ml. 0.1 ml each was inoculated on each well of a 6 well culture plate and subjected to stationary culture at 37°C in the presence of 5% carbon dioxide gas. Twenty four hours after, the culture supernatant was separated by centrifugation (250 x g, 10 minutes) and provided as a sample. ATCC CRL 8658 was cultured in a similar manner to prepare a positive control sample for secreting human gamma chain, human lambda chain and human kappa chain.

A sample for determining an amount of human immunoglobulin synthesized intracellularly in MP 4109 was prepared as follows. The cells in the log phase were collected ($1 \times 10^7$ cells) and suspended in 1 ml of phosphate buffer containing 1% polyoxyethylene sorbitan monolaurate (Sigma) followed by homogenization with a potter type homogenizer. The homogenate was centrifuged (50,000 x g, 30 minutes) and the resulting supernatant was filtered through a filter of 0.22 $\mu$m and provide as a sample. ATCC CRL 8658 was treated in a similar manner to prepare a positive control sample for synthesizing human gamma chain, human lambda chain and human kappa chain. Furthermore, Raji cells were treated in a similar manner to prepare a negative control sample incapable of synthesizing human immunoglobulin. The amounts of human gamma chain, human lambda chain and human kappa chain in the samples were determined by ELISA.

Human gamma chain was not detected in the culture supernatant of MP 4109 and the cell homogenate. The amounts of human lambda chain and human kappa chain secreted by MP 4109 into the culture were all approximately 1/5 that of ATCC CRL 8658. Human gamma chain and human lambda chain were not detected in the culture supernatant of MP 4112 and the cell homogenate. The amounts of human kappa chain secreted by MP 4112 into the culture supernatant were all approximately 1/3 that of ATCC CRL 8658. Human gamma chain, human lambda chain and human kappa chain were not detected in the culture supernatant of MP 4126 and the cell homogenate.

Example 6. Measurement of cell doubling time

MP 4109, MP 4112 and MP 4126 in the log phase were collected and suspended in 10% FCS culture medium in a density of $5 \times 10^4$ cells. 0.1 ml each was inoculated on each well of a 6 well culture plate and subjected to stationary culture at 37°C in the presence of 5% carbon dioxide gas. The cell count in the well was accurately counted once a day using a hemocytometer. The number of the cells in 3 wells was counted every time. The doubling time of MP 4109, MP 4112 and MP 4126 in the log phase determined from the mean value was 24.6, 25.6 and 20.5 hours, respectively.

Example 7. Production of human hybridoma 1:

The culture supernatant of B95-8 cells (marmoset lymphoblast capable of producing infectious EB virus) was added to the human antibody-producing cells (lymphocytes) separated from the peripheral blood of a healthy donor to infect them with EB virus. Then the cells in the well in which the production of human IgM in the culture supernatant was noted were cloned and the resulting human IgM-producing human lymphoblast 87H4G was used as a partner for fusion to produce a human hybridoma. Human IgM-producing human lymphoblast which had been previously proliferated in 10% FCS culture medium and MP 4109 were washed with RPMI 1640 medium, respectively. In a plastic centrifuging tube of 50 ml volume, 3 x $10^7$ human lymphoblast and the same count of MP 4109 cells were mixed with each other. After centrifugation (175 x g, 10 minutes), the culture supernatant was removed by suction and 0.5 ml of RPMI 1640 medium contaianing 50% PEG (molecular weight of 1500, Wako Pure Chemical) and 10% DMSO was gently charged and slowly rotated to cause cell fusion. Two minutes after, 10 ml of RPMI 1640 medium was added. After gently agitating the mixture, centrifugation was carried out (175 x g, 10 minutes). The culture supernatant was removed by suction and 20% FCS culture medium supplemented with 2 x $10^{-4}$ M hypoxanthine (Sigma), 1 $\mu$g/ml azaserine (Sigma) and 5 $\mu$M ouabain (Sigma) (hereafter simply referred to as HA-O medium) was added to the culture supernatant to form a cell suspension in a density of 1 x $10^6$ cells/ml. 0.1 ml each per well of the suspension was inoculated on a 96 well flat bottom culture plate (627 wells in total). The cells were subjected to stationary culture at 37°C in the presence of 5% carbon dioxide gas. Four days after, 0.1 ml of HA-O medium was added and thereafter, half of the HA-O medium was exchanged by a fresh HA-O medium at 4 to 5 day intervals. The number of wells in which proliferation of colonies of human hybridoma was noted until 7 weeks thereafter was 78 out of 627 wells. Frequency in fusion was calculated to be 2.6 x $10^{-6}$.

From the wells in which the proliferation of colonies was noted, 4 wells were chosen at random and human hybridoma was cultured in an increasing scale toward a 24 well plate, a 6 well plate, a 6 cm Petri dish and 75T flask. Four human hybridomas were suspended in 20% FCS culture medium, respectively, in a density of 1 x $10^6$ cells/ml. One ml each per well of the suspension was inoculated on a 6 well plate. The cells were subjected to stationary culture at 37°C in the presence of 5% carbon dioxide gas. Twenty four hours after, centrifugation was performed (200 x g, 10 minutes) and the culture supernatant was separated. The amounts of human mu chain and human gamma chain in the culture supernatant were determined by ELISA. The results are shown in Table 4. All of the human hybridomas secreted human mu chain, which was a heavy chain derived from human IgM-producing human lymphoblast, but did not secrete the other heavy chain (human gamma chain).

Table 4

| Results of Measurement on Amount of Antibody secreted by MP 4109-derived Human Hybridoma | | |
|---|---|---|
| Human Hybridoma | Amount of Antibody Secreted ($\mu$g/$10^6$ cells/24 hrs) | |
| | Human Mu Chain | Human Gamma Chain |
| 2S3B12 | 18.3 | < 0.001 |
| 2S4B7 | 21.1 | < 0.001 |
| 2S7A2 | 33.3 | < 0.001 |
| 2S6B1 | 18.6 | < 0.001 |

Example 8. Production of human hybridoma 2:

In a manner similar to Example 7, 3 x $10^7$ counts of human IgM-producing human lymphoblast 87H4G were fused with the same cells of MP 4112. The number of wells in which proliferation of colonies of human hybridoma was noted until 7 weeks thereafter was 69 out of 610 wells. Frequency in fusion was calculated to be 2.4 x $10^{-6}$.

From the wells in which the proliferation of colonies was noted, 4 wells were chosen at random and human hybridoma was cultured in an increasing scale toward a 24 well plate, a 6 well plate, a 6 cm Petri dish and a 75T flask. Four human hybridomas were suspended in 20% FCS culture medium, respectively, in a density of 1 x $10^6$ cells/ml. One ml each per well of the suspension was inoculated on a 6 well plate. The cells were subjected to stationary culture at 37°C in the presence of 5% carbon dioxide gas. Twenty four hours after, centrifugation was performed (200 x g, 10 minutes) and the culture supernatant was separated. The amounts of human mu chain and human gamma chain in the culture supernatant were determined by ELISA. The results are shown in Table 5. All of the human hybridomas secreted human mu chain, which was a heavy chain derived from human IgM-producing human lymphoblast 87H4G but did not secrete other heavy chain (human gamma chain).

Table 5

| Results of Measurement on Amount of Antibody Secreted by MP 4112-derived Human Hybridoma | | |
|---|---|---|
| Human Hybridoma | Amount of Antibody Secreted ($\mu$g/$10^6$ cells/24 hrs) | |
| | Human Mu Chain | Human Gamma Chain |
| 3N1A8 | 25.6 | < 0.001 |
| 3N3C6 | 15.8 | < 0.001 |
| 3N3E12 | 28.2 | < 0.001 |
| 3N5A3 | 35.5 | < 0.001 |

Example 9. Production of human hybridoma 3:

The culture supernatant of B95-8 cells was added to the human antibody producing cells (lymphocytes) separated from the peripheral blood of a healthy donor to infect with EB virus. Then the cells in the well in which the production of human IgM in the culture supernatant was noted were cloned and the resulting human IgM (human mu chain and lambda chain)-producing human lymphoblast 87L8GNM was produced.

In a manner similar to Example 7, 2.5 x $10^7$ cells of 87L8GNM was fused with the same counts of MP 4126. The number of wells in which proliferation of colonies of human hybridoma was noted until 7 weeks thereafter was 6 out of 480 wells. As the result, frequency in fusion was calculated to be 2.4 x $10^{-7}$.

From the wells in which the proliferation of colonies was noted, 4 wells were chosen at random and human hybridoma was cultured in an increasing scale toward a 24 well plate, a 6 well plate, a 6 cm Petri dish and a 75T flask. Four human hybridomas were suspended in 20% FCS culture medium, respectively, in a density of 1 x $10^6$ cells/ml. One ml each per well of the suspension was inoculated on a 6 well plate. The cells were subjected to stationary culture at 37°C in the presence of 5% carbon dioxide gas. Twenty four hours after, centrifugation was performed (200 x g, 10 minutes) and the culture supernatant was separated. The amount of human mu chain and the amounts of human gamma chain in the culture supernatant were determined by ELISA. The results are shown in Table 6. All of the human hybridomas secreted only human mu chain which were heavy chain derived from human IgM-producing human lymphoblast, but secreted no other heavy chain (human gamma chain). The amount of human kappa chain and human lambda chain in the culture supernatant were determined by ELISA. Human lambda chain was detected, but human kappa chain was not detected.

EP 0 368 662 B1

Table 6

| Results of Measurement on Amount of Antibody Secreted by MP 4126-derived Human Hybridoma | | |
|---|---|---|
| Human Hybridoma | Amount of Antibody Secreted ($\mu$g/$10^6$ cells/24 hrs) | |
| | Human Mu Chain | Human Gamma Chain |
| 4C1H9 | 17.8 | <0.001 |
| 4C2F1 | 21.3 | <0.001 |
| 4C2G6 | 30.6 | <0.001 |
| 4C3B12 | 12.2 | <0.001 |

EFFECTS OF THE INVENTION

By using the novel parent cell line of the present invention, human hybridomas with human antibody-producing cells can be produced.

What is notable in the present invention is that the human hybridomas produced by cell fusion between the novel parent cell lines of the present invention, MP 4109, MP 4112 and MP 4126, and human antibody-producing cells contain no human immunoglobulin heavy chain derived from the parent cell lines at all in the resulting human monoclonal antibodies. It is particularly of great significance in the case of industrially utilizing human monoclonal antibodies produced by human hybridomas that a parent cell line for producing human hybridomas do not synthesize human immunoglobulin heavy chain.

The human hybridomas produced by cell fusion between the heavy chain-non-producing parent cell line of the present invention and human antibody-producing cells of the IgA type, IgG type, IgM type, IgE type or IgD type do not synthesize heavy chain derived from the parent cell lines so that the desired antibodies of the IgA type, IgG type, IgM type, IgE type or IgD type can be readily purified. The human hybridomas produced by cell fusion between the heavy chain-non-producing parent cell line of the present invention and human antibody-producing cells are free of any chance to produce antibodies in which heavy chain derived from the parent cells is recombined with the desired antibodies. Furthermore, the human hybridomas produced by cell fusion between MP 4126 and antibody-producing cells synthesize only human immunoglobulin derived from human antibody-producing cells so that the desired antibodies can be readily purified. The use of the novel parent cell lines of the present invention can be appropriately selected depending upon the Type of human antibody-producing cells or purpose of using the antibodies produced.

The parent cell line of the present invention can be fused with a variety of human antibody-producing cells of different origins. After the cell fusion, the human hybridomas produced can be selectively proliferated by their own selectivity characteristics. As the human antibody-producing cells, there may be used, for example, antibody-producing cells isolated from spleen, lymph node, tonsil, peripheral blood, etc. of healty donor or patients with various diseases; cell masses obtained by transforming these antibody-producing cells with EB virus and single EB virus transformant obtained from these transformed cell masses by cloning. Furthermore, human antibody-producing cells in which a high blood antibody titer to a specific antigen is induced by immunization with a specific antigen, antibody-producing cells obtained by stimulating and proliferating by culturing human antibody-producing cells (B cells) in a medium supplemented with factors such as poke weed mitogen (PWM), etc. and a specific antigen for several days, and the like, may also be used as the human antibody-producing cells for cell fusion.

By cell fusion between the novel parent cell lines of the present invention and human antibody-producing cells, human monoclonal antibodies which can be used for diagnosis, prophylaxis and treatment of various diseases can be produced. For example, it is now possible to produce human monoclonal antibodies to viruses such as cytomegalovirus (CMV), human T cell leukemia virus (HTLV), herpes simplex virus (HSV), varicella-zoster virus (VZV), hepatitis B virus (HBV), influenza virus, RS virus (RSV), etc.; bacteria such as Pseudomonas aeruginosa, pathogenic Escherichia coli, Haemophilus influenzae, Diplococcus pnumoniae, Staphylococcus aureus, etc.; fungi such as Aspereirus and Candida. Human monoclonal antibodies which are obtainable by this invention can be utilised in a wide range of applications such as classification of cells, electrophoretic analysis, purification of substances, histology, cellology, etc., in addition to diagnosis and therapy. For example, thanks to the invention, human monoclonal antibodies to cancer antigen, toxin such as exotoxin A, etc. produced by Pseudomonas aeruginosa, various allergens such as cedar pollens, hormones, physiologically active proteins, and histocompatible antigens are all now possible. The globulin class of antibodies produced by the human

14

EP 0 368 662 B1

antibody-producing cells may be any one of the various isotypes including IgG, IgM, IgA, IgD and IgE.

Furthermore, the parent cell line of the present invention can be used not only as the parent cell line for cell fusion but also as host cells for producing various proteins by transfecting and expressing various genes. In addition, the parent cell line can also be used as cells for preparing human antibody genes by producing human hybridomas with human antibody-producing cells.

The novel parent cell line of the present invention can impart a high antibody-secreting ability to the human hybridomas produced by cell fusion. By this property, the time period for incubation can be shortened and production costs can be reduced in the case of producing human monoclonal antibodies through culturing the human hybridomas produced in large quantities.

Either by selecting the parent cell line of the present invention or by selecting after induction of mutation, a new human immunoglobulin-non-producing cell line can be obtained. Alternatively, the parent cell line of the present invention can be fused with human myeloma cells or human lymphoblasts to create a new parent cell line. The parent cell line of the present invention can also be directed to a new parent cell line by introducing a foreign gene for imparting selectivity characteristics or to induce mutation. For example, a plasmid having a gene resistant to chemicals such as neomycin, etc. can be introduced into the parent cell line or the parent cell line can be fused with cells having a gene resistant thereto.

**Claims**
**Claims for the following Contracting States : CH, DE, FR, GB, IT, LI, NL, SE**

1. A human γ-chain-non-synthesizing mutant cell line derived from human immunoglobulin-synthesizing cell line ATCC CRL 8658, and suitable for use as a fusion partner for making human hybridomas capable of synthesizing specific monoclonal antibodies, and a functionally-equivalent cell line derived therefrom.

2. A mutant cell line of Deposit No. FERM BP-2128, FERM BP-2129 or FERM BP-2615, and a functionally-equivalent cell line derived therefrom.

3. Use of the mutant cell line according to claim 1 or claim 2, as a fusion partner for making human hybridomas with human antibody-producing cells.

4. A human hybridoma obtained by cell fusion between the mutant cell line according to claim 1 or claim 2, and human antibody-producing cells, and a functionally-equivalent cell line derived therefrom.

5. A human hybridoma obtained by fusing cells of the mutant cell line of Deposit No. FERM BP-2128, FERM BP-2129 or FERM BP-2615 and human antibody-producing cells.

6. The invention of claim 3, 4 or 5, wherein the human antibody-producing cells are selected from cells isolated from the spleen, lymph node, tonsils and peripheral blood of healthy or infected donors, or such cells transformed with EB virus, or human antibody-producing cells which have been induced by immunization to produce antibody to a specific antigen.

7. Monoclonal antibodies from the hybridomas of claim 4, 5 or 6.

8. Use of the human hybridomas according to claim 4, 5 or 6 for the production of a monoclonal antibody for use in diagnosis, prophylaxis and treatment of diseases.

9. Use of the human hybridomas according to claim 4, 5 or 6 for the production of monoclonal antibodies to:
   (i) viruses including cytomegalovirus, human T cell leukaemia virus, herpes simplex virus, varicella-zoster virus, hepatitis B virus, influenza virus, RS virus;
   (ii) bacteria including Pseudomonas aeruginosa, pathogenic E. coli, Haemophilus influenzae, Diplococcus pnumoniae, Staphylococcus aureus;
   (iii) fungi including Aspergirus and Candida; and
   (iv) cancer antigen, toxins such as exotoxin A, allergens, hormones, physiologically active proteins and histocompatible antigens.

15

**Claims for the following Contracting State : ES**

1. A method of making a human hybridoma comprising fusing a human γ-chain-non-synthesizing mutant cell line derived from human immunoglobulin-synthesising cell line ATCC CRL 8658, and human antibody-producing cells.

2. A method according to claim 1, wherein the mutant cell line is the mutant cell line of Deposit No. FERM BP-2128, FERM BP-2129 or FERM BP-2615, and a functionally-equivalent cell line derived therefrom.

3. The method of claim 1 or claim 2, wherein the human antibody-producing cells are selected from cells isolated from the spleen, lymph node, tonsils and peripheral blood of healthy or infected donors, or such cells transformed with EB virus, or human antibody-producing cells which have been induced by immunization to produce antibody to a specific antigen.

4. Use of the human hybridomas produced by the method according to claim 1, 2 or 3 for the production of a monoclonal antibody for use in diagnosis, prophylaxis and treatment of diseases.

5. Use of the human hybridomas prepared by the method according to claim 1, 2 or 3 for the production of monoclonal antibodies to:
    (i) viruses including cytomegalovirus, human T cell leukaemia virus, herpes simplex virus, varicella-zoster virus, hepatitis B virus, influenza virus, RS virus;
    (ii) bacteria including Pseudomonas aeruginosa, pathogenic E. coli, Haemophilus influenzae, Diplococcus pneumoniae, Staphylococcus aureus;
    (iii) fungi including Aspergirus and Candida; and
    (iv) cancer antigen, toxins such as exotoxin A, allergens, hormones, physiologically active proteins and histocompatible antigens.

**Patentansprüche**
**Patentansprüche für folgende Vertragsstaaten : CH, DE, FR, GB, IT, LI, NL, SE**

1. Eine humane γ-Ketten-nicht-synthetisierende Mutantenzellinie erhalten aus einer humanen Immunoglobulin synthetisierenden Zellinie ATCC CRL 8658 und geeignet als Fusionspartner zur Herstellung von humanen Hybridomas, die in der Lage sind, spezifische monoklonale Antikörper zu synthetisieren, und eine daraus abstammende funktionell equivalente Zellinie.

2. Eine Mutantenzellinie der Hinterlegungsnummer FERM BP-2128, FERM BP-2129 oder FERM BP-2615 und eine daraus abstammende funktionell equivalente Zellinie.

3. Verwendung der Mutantenzellinie gemäß Patentanspruch 1 oder Patentanspruch 2 als Fusionspartner zur Herstellung von humanen Hybridomas mit humanen Antikörper produzierenden Zellen.

4. Eine humane Hybridoma erhalten durch Zellfusion zwischen der Mutantenzellinie gemäß Patentanspruch 1 oder Patentanspruch 2 und humanen Antikörper produzierenden Zellen und eine daraus abstammende funktionell equivalente Zellinie.

5. Eine humane Hybridoma erhalten durch Fusion von Zellen der Mutantenzellinie der Hinterlegungsnummer FERM BP-2128, FERM BP-2129 oder FERM BP-2615 und humanen Antikörper produzierenden Zellen.

6. Die Erfindung der Patentansprüche 3, 4 oder 5, worin die humane Antikörper produzierenden Zellen ausgewählt sind aus Zellen isoliert aus Milz, Lymphknoten, handeln und peripheralem Blut von gesunden oder infizierten Spendern, oder solche Zellen transformiert mit EB-Virus oder humane Antikörper produzierenden Zellen, die durch Immunisierung veranlaßt wurden, Antikörper zu einem spezifischen Antigen zu produzieren.

7. Monoklonale Antikörper aus den Hybridomas gemäß den Patentansprüchen 4, 5 oder 6 .

**8.** Verwendung der humanen Hybridomas gemäß den Patentansprüchen 4,5 oder 6 für die Herstellung monoklonaler Antikörper zur Verwendung in der Diagnose, Prophylaxe und Behandlung von Krankheiten.

**9.** Verwendung der humanen Hybridomas gemäß den Patentansprüchen 4, 5 oder 6 für die Produktion von monoklonalen Antikörpern zu:

(I) Viren einschließlich Cytomegaloviren, humanen T-Zellen-Leukämie-Viren, Herpes Simplex Viren, Varizella-Zoster-Viren, Hepatitis B-Viren, Grippe Viren, RS-Viren;

(II) Bakterien einschließlich Pseudomonas aeruginosa, pathogene E. coli, Haemophilus influenzae, Diplococcus pnumoniae, Staphylococcus aureus;

(III) Pilze einschließlich Aspergirus und Candida; und

(IV) Krebs-Antigen, Toxine wie Exotoxin A, Allergene, Hormone, physiologisch aktive Proteine und gewebeverträgliche Antigene.

**Patentansprüche für folgenden Vertragsstaat : ES**

**1.** Ein Verfahren zur Herstellung eines humanen Hybridoma, gekennzeichnet durch Fusion einer humanen $\gamma$-Kettennicht-synthetisierenden Mutantenzellinie, erhalten aus einer humanen Immunoglobulin synthetisierenden Zellinie ATCC CRL 8658, und humanen Antikörper produzierenden Zellen.

**2.** Ein Verfahren gemäß Patentanspruch 1, worin die Mutantenzellinie die Mutantenzellinie der Hinterlegungsnummer FERM BP-2128, FERM BP-2129 oder FERM BP-2615 und eine daraus abstammende, funktionell equivalente Zellinie ist.

**3.** Das Verfahren gemäß Patentanspruch 1 oder Patentanspruch 2, worin die humanen Antikörper produzierenden Zellen ausgewählt sind aus Zellen isoliert aus Milz, Lymphknoten, handeln und peripheralem Blut von gesunden oder infizierten Spendern, oder solche Zellen transformiert mit dem EB-Virus oder humane Antikörper produzierende Zellen, die durch Immunisierung veranlaßt wurden, Antikörper zu einem spezifischen Antigen zu produzieren.

**4.** Verwendung der humanen Hybridomas produziert durch das Verfahren gemäß Patentanspruch 1,2 oder 3 zur Herstellung eines monoklonalen Antikörpers zur Verwendung in der Diagnose, Prophylaxe und Behandlung von Krankheiten.

**5.** Verwendung der humanen Hybridomas hergestellt durch die Methode gemäß Patentanspruch 1, 2 oder 3 zur Produktion von monoklonalen Antikörpern zu:

(I) Viren einschließlich Cytomegaloviren, humanen T-Zellen-Leukämie-Viren, Herpes Simplex-Viren, Varizella-Zoster-Viren, Hepatitis B-Viren, Grippe-Viren, RS-Viren;

(II) Bakterien einschließlich Pseudomonas aeruginosa, pathogene E. coli, Haemophilus influenzae, Diplococcus pnumoniae, staphylococcus aureus;

(III) Pilze einschließlich Aspergirus und Candida; und

(IV) Krebs-Antigen, Toxine wie Exotoxin A, Allergene, Hormone, physiologisch aktive Proteine und gewebeverträgliche Antigene.

**Revendications**

**Revendications pour les Etats contractants suivants : CH, DE, FR, GB, IT, LI, NL, SE**

**1.** Lignée cellulaire humaine mutante ne synthétisant pas la chaîne $\gamma$ dérivée de la lignée cellulaire humaine ATCC CRL 8658 synthétisant des immunoglobulines, utilisable en tant que partenaire de fusion dans la préparation d'hybridomes humains capables de synthétiser des anticorps monoclonaux spécifiques et une lignée cellulaire équivalente de cette dernière d'un point de vue fonctionnel.

**2.** Lignée cellulaire mutante déposée sous le numéro FERM BP-2128, FERM BP-2129 ou FERM BP-2615, et une lignée cellulaire issue de cette dernière équivalente d'un point de vue fonctionnel.

**3.** Utilisation de la lignée cellulaire mutante définie à la revendication 1 ou 2 en tant que partenaire de fusion pour préparer des hybridomes humains avec des cellules humaines productrices d'anticorps.

**4.** Hybridome humain obtenu par fusion cellulaire entre la lignée cellulaire définie à la revendication 1 ou 2 et des cellules humaines productrices d'anticorps et une lignée cellulaire issue de cette dernière équivalente d'un point de vue fonctionnel.

**5.** Hybridome humain obtenu par fusion de cellules de la liée cellulaire mutante déposée sous le numéro FERM BP-2128, FERM BP-2129 ou FERM BP-2615 et des cellules humaines productrices d'anticorps.

**6.** Invention selon l'une des revendications 3, 4 ou 5 dans laquelle les cellules humaines productrices d'anticorps sont choisies parmi des cellules isolées à partir de la rate, des ganglions lymphatiques, des amygdales ou du sang périphérique de donneurs sains ou infectés ou des cellules de ce tue transformées par le virus EB, ou des cellules productrices d'anticorps qui ont été induites à produire un anticorps contre un antigène spécifique par immunisation.

**7.** Anticorps monoclonaux issus des hybridomes des revendications 4, 5 ou 6.

**8.** Utilisation des hybridomes humains selon la revendication 4, 5 ou 6 pour produire un anticorps monoclonal à usage diagnostic, prophylactique et thérapeutique de maladies.

**9.** Utilisation des hybridomes humains selon la revendication 4, 5 ou 6 pour produire des anticorps monoclonaux dirigés contre:
(i) des virus incluant le cytomégalovirus, le virus de la leucémie à lymphocytes T humains, le virus de l'herpès simplex, le virus de la varicelle, le virus de l'hépatite B, le virus de la grippe, le virus RS;
(ii) des bactéries incluant Pseudomonas aeruginosa, les souches pathogènes d'E. coli, Haemophilus influenzae, Diplococcus pneumoniae et Staphylococcus aureus;
(iii) des champignons incluant Aspergirus et Candida; et
(iv) des antigènes associés au cancer, des toxines tels que l'exotoxine A, des allergènes, des hormones et des protéines actives sur le plan physiologique ainsi que des antigènes histocompatibles.

**Revendications pour l'Etat contractant suivant : ES**

**1.** Procédé de préparation d'un hybridome humain comprenant la fusion d'une lignée cellulaire mutante humaine ne synthétisant pas la chaîne $\gamma$ dérivée de la lignée cellulaire ATCC CRL 8658 synthétisant des immunoglobulines et des cellules humaines produisant des anticorps.

**2.** Procédé selon la revendication 1, dans lequel la lignée cellulaire mutante est la lignée cellulaire mutante déposée sous le numéro FERM BP-2128, FERM BP-2129 ou FERM BP-2615, et une lignée cellulaire issue de cette dernière équivalente d'un point de vue fonctionnel.

**3.** Procédé selon la revendication 1, ou 2, dans lequel les cellules humaines productrices d'anticorps sont choisies parmi des cellules isolées à partir de la rate, des ganglions lymphatiques, des amygdales ou du sang périphérique de donneurs sains ou infectés ou des cellules de ce type transformées par le virus EB, ou des cellules humaines productrices d'anticorps qui ont été induites à produire un anticorps contre un antigène spécifique par une immunisation.

**4.** Utilisation des hybridomes humains produits par le procédé selon la revendication 1, 2 ou 3 pour produire un anticorps monoclonal destinés à un usage diagnostic, prophylactique ou thérapeutique de maladies.

**5.** Utilisation des hybridomes humains préparés à l'aide du procédé selon la revendication 1, 2 ou 3 pour produire des anticorps monoclonaux dirigés contre:
(i) des virus incluant le cytomégalovirus, le virus de la leucémie humaine à lymphocytes T, le virus de l'herpès simplex, le virus de la varicelle, le virus de l'hépatite B, le virus de la grippe, le virus RS;
(ii) des bactéries incluant Pseudomonas aeruginosa, les souches pathogènes d'E. coli, Haemophilus influenzae, Diplococcus pneumoniae et Staphylococcus aureus;
(iii) des champignons incluant Aspergirus et Candida; et
(iv) des antigènes associés au cancer tels que l'exotoxine A, des allergènes, des hormones et des protéines actives au plan physiologique ainsi que des antigènes histocompatibles.